# EUROPEAN PATENT SPECIFICATION

(11) **EP 2 623 064 B1**
(45) Date of publication and mention of the grant of the patent: **12.11.2014**
(21) Application number: 11788907.1
(22) Date of filing: 22.09.2011
(51) Int. Cl.: A61C 8/00, A61B 17/66

(54) **IMPLANT FOR EXPANDING THE JAWBONE CREST**
IMPLANTAT ZUM SPREIZEN DES KIEFERKNOCHENKAMMS
IMPLANT POUR ÉLARGIR LA CRÊTE OSSEUSE DE LA MÂCHOIRE

(30) Priority: 30.09.2010 ES 201001258
(43) Date of publication of application: 07.08.2013
(73) Proprietor: Biotechnology Institute, I Mas D, S.L., 01005 Vitoria (Álava) (ES)
(72) Inventor: ANITUA ALDECOA, Eduardo, E-01005 Vitoria (ES)
(74) Representative: Urteaga Simarro, José Antonio
(86) International application number: PCT/ES2011/000286
(87) International publication number: WO 2012/042071

(56) References cited:
- EP-A2- 1 348 387
- WO-A1-03/073955
- WO-A2-2005/018684
- TOGNARINI ET AL: "In vitro differentiation of human mesenchymal stem cells on Ti6Al4V surfaces", BIOMATERIALS, ELSEVIER SCIENCE PUBLISHERS BV., BARKING, GB, vol. 29, no. 7, 26 November 2007 (2007-11-26), pages 809-824, XP022394090, ISSN: 0142-9612, DOI: 10.1016/J.BIOMATERIALS.2007.10.043

## Description

### Technical field

The invention relates to an implant-expander for expanding bone crest, i.e. for expanding or widening the bone crest of the jawbone of a patient in the event that said crest is too narrow for it to host a dental implant correctly.

### Prior art

A dental implant, as is known, is a piece designed to be placed in the jawbone of a patient and to act as a support for the connection of a dental prosthesis or artificial tooth. A dental implant positioned in the jawbone of a patient undergoes a process known as osseointegration, which results in the structural and functional connection between the bone cells of the living bone surrounding the dental implant and the surface of the dental implant itself. Several months must elapse from the insertion of the implant in the jawbone for the osseointegration of the implant to take place.

Osseointegration between the implant and the surrounding bone takes place thanks to certain properties of the dental implants. On one hand, the implant is manufactured from a biocompatible material, i.e. a material that is capable of being implanted in a living being. In addition, the implant is manufactured from a material that is also osteoconductive, in other words, the implant has the capacity to act as a passive framework that supports the new formation and growth of the bone. The most commonly used biocompatible and osteoconductive material in the manufacture of implants is titanium. Titanium also has mechanical properties similar to that of bone, which means that when exposed to loads, it suffers deformations similar to those suffered by bone, preventing relative microdeformations between the bone and the implant, which would negatively affect the bone remodelling rate and therefore reduce osseointegration.

The mechanical design of the dental implant may also encourage its osseointegration. Implants thus generally have an optimised body and external thread to prevent, under axial loads, the implant from transmitting torsional stress and shearing to the bone, which negatively affect the bone-implant interphase in terms of bone remodelling. In addition, it is important that the external thread of the implant is such that tension peaks do not occur between the first rings of said external thread and the bone, which may cause the bone to recede. In fact, the external thread must be designed in such a way that it transmits moderate tensions that are beneficial to the bone remodelling process. Furthermore, at micrometric level the surface of the implant that is in contact with the bone may be increased by means of procedures such as the acid treatment described in EP1352665B1, awarded to the applicant, which increases the osseointegration. In addition, at a micrometric level there are also procedures such as the acid treatment described in EP1352665B1, which make the surface hydrophilic, in other words it attracts human fluids (blood, plasma rich in platelets, etc) in the initial phases of osseointegration, encouraging it.

Dental implants are positioned in an elongated protuberance of the jawbone known as bone crest. The bone crest may on occasion be too narrow to receive a dental implant, it therefore being necessary to carry out a bone crest expansion process in order to widen the bone crest. A process of this type is described in patent application WO2004019807, which explains that the bone crest is expanded by means of the drilling of a narrow initial alveolus in the bone crest, which is subsequently expanded by means of the successive insertion of bone crest expanders of increasing width, used in combination with drills that increase the diameter of the alveolus in order to prepare it to receive the subsequent bone crest expander. When the alveolus acquires the necessary size and the bone crest is sufficiently wide, a bone regeneration procedure is usually executed by means of the application of Platelet-Rich Plasma (PRP) (for example, plasma rich in growth factors (PRGF) obtained according to patent EP1066838). Finally, an entirely new cavity or alveolus is drilled in the widened bone crest, said new cavity being the one that houses the final dental implant.

Expanders used in prior art are tools generally manufactured from surgical-grade stainless steel, with the result that they may be sterilised in an autoclave and are not cytotoxic, as a result of which they may be used as a surgical instrument to carry out operations in which they are required to be in contact with living tissue for several minutes.

WO2005/018 684 describes an implant-expander for expanding the bone crest of a patient for the purpose of receiving the insertion of a final dental implant. The implant-expander is manufactured from a biocompatible and osteoconductive material It comprises a threaded body, a crown area and an apex. The crown area has an outer diameter in continuity with that of the threaded body and is not wider than the threaded body. The crown area comprises an anti-rotational blind hole for the insertion of a torque applying tool.
It is an object of the invention to provide an alternative implant expander for expanding bone crest, which offers a series of biological and medical advantages over the method described above. These advantages include being able to perform a widening of bone crest in which the bone crest presents regenerated bone of a higher quality and improved vascularisation; this effect results in the improved initial stability of the final dental implant or even enables a subsequent expansion (known as accordion effect) to be carried out.

### Brief description of the invention

It is an object of this invention to provide an implant-expander, for expanding the bone crest of a patient for the purpose of receiving the insertion of a final dental implant. The implant-expander is manufactured from a biocompatible and osteoconductive material. It comprises a threaded body, a crown area and an apex. The crown area has an outer diameter in continuity with that of the threaded body and is not wider than the threaded body. The crown area comprises an anti-rotational blind hole for the insertion of a torque applying tool. The crown area has outer walls that are partly threaded. The outer walls of the crown area have increasingly deep thread characteristics until they match those of the thread of the threaded body.

The implant-expander according to the invention is a part that has biocompatible and osteoconductive properties that give it the capacity to osseointegrate with the surrounding bone crest. In addition, it may optionally have other characteristics that encourage osseointegration, such as surface roughness or hydrophilicity obtained following an acid treatment, a threaded body provided with an optimised design, etc.

One of the main advantages of the invention is that the implant-expander used to widen the bone crest acts as a support to the bone and therefore helps consolidate the fracture of the bone crest (the bone crest is fractured so that it may be widened) and helps vascularised bone to be formed around the implant-expander. In other words, high-quality (vascularised) bone is generated around the implant-expander, whereas based on the use of conventional expanders bone is not generated around the expander, with the expanders merely widening the fracture formed in the bone crest.

Another important advantage of the invention is that it enables larger widenings to be achieved due to the fact it is possible to perform successive widening phases thanks to the widening process taking place at the same time as the generation of high-quality bone around the implant-expander. Thus, when the implant-expander is removed, the already partially widened bone crest may receive an additional widening phase, according to the same or other procedures.

Another significant advantage of the invention is that it allows the angulation of the bone crest to be corrected, which is useful in situations in which the final dental implant needs to be oriented in a different direction to the one offered the bone crest. In these situations, due to the fact that when the implant-expander is removed the bone that remains is of a high quality, it is possible to open a new alveolus in a different direction to that of the initial implant-expander, and then insert the final dental implant or continue with another expansion process. Expressed in different terms, the method according to the invention allows, if required in practice, to correct the angulation of the alveolus inside the bone crest so that the final dental implant is oriented in the correct direction to enable a correct orientation of the final dental prosthesis.

Another advantage, which is a result of the preceding ones, is that the invention allows the widening of bone crests that are initially very narrow (2.5 mm wide) and which are impossible to treat with the conventional widening technique by means of expanders. This is made possible by two factors: firstly, by using a sufficiently narrow implant-expander; secondly, because said implant-expander is capable of generating high-quality bone around itself, filling the fracture of the bone crest. Then, the narrow bone crest can be expanded in two phases, as a result of which expansions of up to 200% can be achieved.

An additional advantage of the invention is its predictable nature, which is especially beneficial in expanding narrow crests, the expansion of which has to date yielded overly unpredictable or random results.

### Brief description of the drawings

Details of the invention can be seen in the accompanying non-limiting drawings:
- Figures 1a to 1j show an example a method used with the invention.
- Figures 2 and 3 show a cross-sectional elevation and a top view of an embodiment of an implant-expander.
- Figures 4 and 5 show a cross-sectional elevation and a top view of an embodiment of an implant-expander according to the invention.
- Figures 6 and 7 show a cross-sectional elevation and a top view of an implant-expander.
- Figures 8 and 9 show a cross-sectional elevation and a top view of a second embodiment of an implant-expander according to the invention.

### Detailed description of the invention

The invention proposes an implant-expander for expanding the bone crest of a patient so that it can receive the insertion of a final dental implant. The formation in the bone crest of an alveolus that is subsequently widened. A specific feature of the the invention is that, in order to widen the alveolus and the bone crest, an implant-expander provided with biocompatible and osteoconductive properties is inserted in said alveolus. When the implant-expander occupies the alveolus, osseointegration of the implant-expander takes place due to its biocompatible and osteoconductive properties. As a result, using a piece with implant properties in order to widen the bone crest allows two effects to be achieved: firstly, the widening itself is achieved; secondly, the bone crest fracture is consolidated, with vascularised bone being formed around the implant-expander, said bone being of a very high quality even in very narrow bone crests (of a width of 2.5 mm) that cannot be widened by conventional techniques. This entails a number of additional advantages that shall be explained at a later stage.

Preferably, the implant-expander is manufactured from titanium, and in an especially advantageous manner, from grade 5 titanium (Ti₆Al₄V). Grade 5 titanium does not have such a high biocompatibility as pure titanium (CP4) but it does have mechanical properties that are more similar to those of the bone. As a result, it is deemed ideal for use in the manufacture of a piece designed for the method according to the invention, which is to be installed in the bone in a non-permanent manner.

Preferably, the implant-expander made of titanium has a rough surface obtained by surface treatment with acids. This ensures that only bone is formed around the implant-expander and that no connective tissue is formed.

Once the bone crest has been widened and the fracture consolidated, the implant-expander is removed. In order to carry out its removal as atraumatically as possible, a tool and a procedure of the type described in patent application WO2009153372, awarded to the applicant, may, for example, be used. This procedure allows removing the implant-expander without altering the surrounding bone and requiring the application of a very low counter-torque in 100 per cent of cases. When the implant-expander is removed, by way of example, if the expansion has been performed on a bone crest with an initial width of 2.5 mm, it is calculated that the bone crest is widened to 5 mm.

The alveolus that remains following removal of the implant-expander has a size substantially equal to that of the removed implant and may be practically prepared (it will require light drilling) for installing the final dental implant, of a larger diameter.

More than one implant-expander may be used, generally of an increasing diameter, in a successive manner. This will enable the gradual widening of the bone crest and allow the formation of even larger widenings.

Depending on the clinical case under treatment, the angulation of the alveolus inside the bone crest is corrected as it is widened. In other words, in the case of a single implant-expander being used, and a final dental implant subsequently being inserted, the implant-expander is inserted in an initial direction and the final dental implant inserted in a different direction to the initial direction. In addition, in the case of various widening implants being used in a successive manner, the direction of insertion of the widening dental implants is not fixed, in other words that the angulation of the alveolus is corrected so that the final dental implant is properly oriented. This correction of the angulation, which in practice is needed to be carried out on a very frequent basis, can be carried out thanks to the fact the initial implant-expander has generated high-quality vascularised bone around it.

Preferably, the implant-expander is provided with a threaded area with a diameter ranging between 2 and 3.5 mm, in other words with a diameter that is very small. This allows a gradual and risk-free widening process, which is especially useful for very narrow bone crests (for example, 2.5-3 mm wide) which cannot be treated in a predictable manner by conventional techniques, in which bone is always lost in the top part as the vascular supply does not reach that area.

Optionally, particulate grafts may be applied on the bone crest along with each implant-expander in order to overcorrect the shape of the bone crest besides producing its widening. It is also possible to apply Platelet-Rich-Plasma (PRP) on the bone crest and/or the alveolus, said Platelet-Rich-Plasma (PRP) preferably comprising plasma rich in growth factors (PRGF) obtained, for example, according to the method of patent EP1066838. The addition of PRP/PRGF to the alveolus encourages coagulation and the osseointegration of implant-expander in the bone, as well as the consolidation of the fracture of the bone crest caused by the expansion.

The use of dental implants to enable the widening of bone crest has some advantages:
- Wider bone crests may be obtained than with the conventional procedure, as the bone crest is widened in a more gradual manner (this is because the method according to the invention is preferably performed with implants-expanders of a small diameter). This reduces the risk of breaking the walls of the bone crest and also improves vascularisation.
- The final dental implant may be positioned in more varied directions, thanks to the fact that the axiality of the alveolus is varied in order to direct it in the direction in which the final dental implant and the corresponding dental prosthesis are to be positioned. Correcting the axiality is possible because a greater widening of the bone crest is allowed, together with a higher-quality bone (vascularised bone).

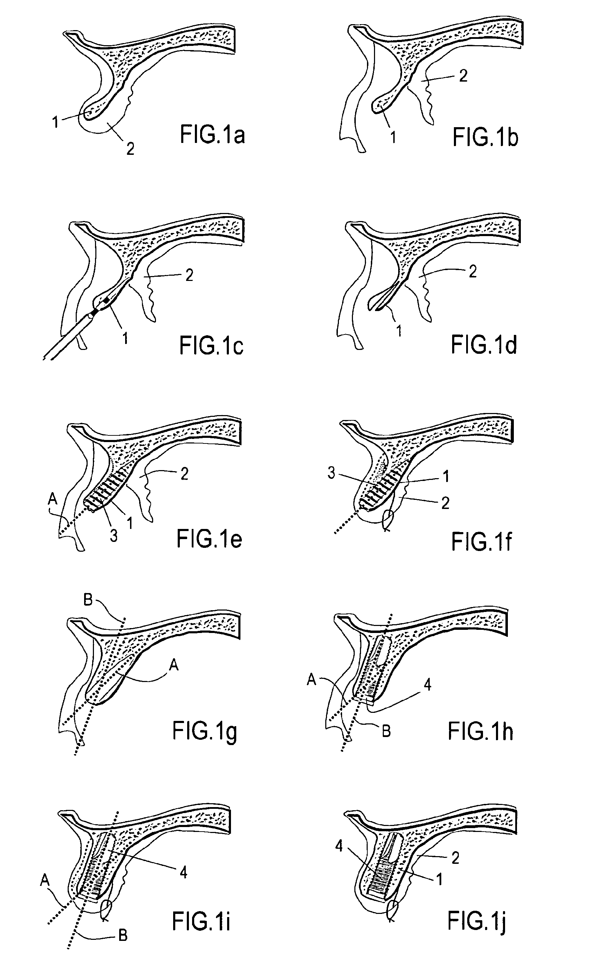

An example of the execution of the procedure can be seen in the sequence of steps detailed in Figures 1a to 1j. Figure 1a shows an initial bone crest (1) covered by the gum (2). After the gum (2) is opened, as shown in Figure 1b, an incision is made in the bone crest (1), as shown in Figure 1c, in order to create an initial hole or alveolus, as shown in Figure 1d. An implant-expander (3) is then inserted into said initial alveolus according to a direction (A), as shown in Figure 1e. As shown in Figure If, a particulate graft is then applied in order to overcorrect the bone crest, the gum (2) is then closed and sutured, the implant-expander (3) thus becoming buried. Once the implant-expander (3) has osseointegrated and the fracture of the bone crest has consolidated (with the implant-expander acting as a support), the implant-expander (3) is removed in an atraumatic manner, leaving a hole, as shown in Figure 1g. In this situation, the bone crest has been widened to even twice its original width. Then, as shown in Figure 1h, after light drilling a final dental implant (4) is inserted in a different direction (B) to the direction (A) of the implant-expander (3), as it is in said new direction (B) that the final dental implant (4) ought to be positioned so that the final dental prosthesis is correctly oriented in the mouth of the patient. Then, particulate graft material is added as shown in Figure 1i, and the gum (2) is closed and sutured. Figure 1j shows the final state of the bone crest (1), significantly widened and with the final dental implant (4) having been installed.

The invention proposes a specific implant-expander that is especially suitable for executing such a method. Said implant-expander is manufactured, as stated, from a biocompatible and osteoconductive material, preferably from titanium and, in an especially advantageous manner, from grade 5 titanium (Ti₆Al₄V).

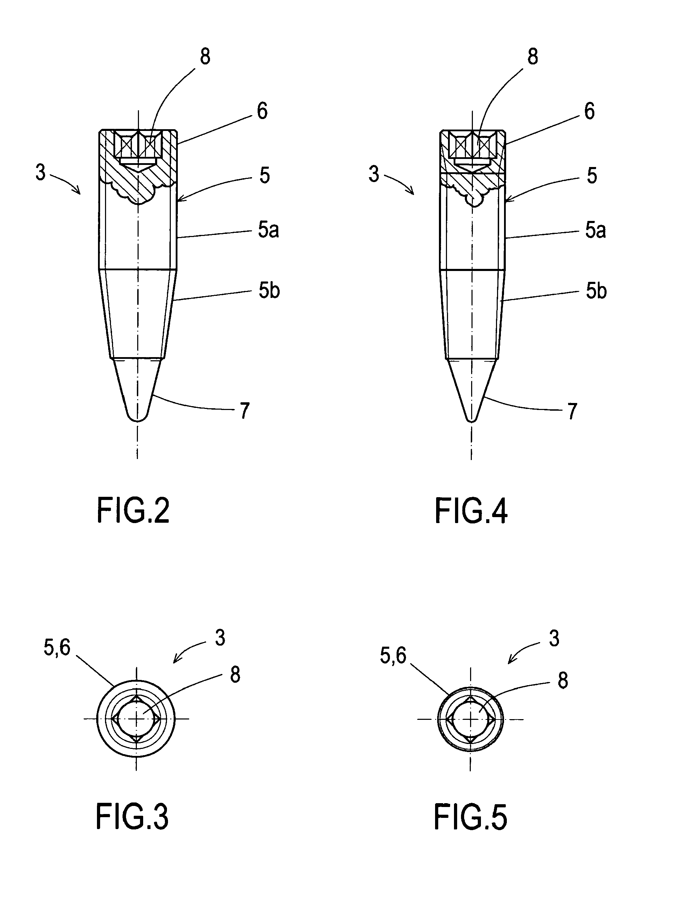

Figures 2 and 3 show an implant-expander. As can be seen, the implant-expander (3) comprises, as do other conventional dental implants, a threaded body (5), a crown area (6) and an apex (7). However, the inventive implant has certain notable specific features that make it ideal for enabling the widening of bone crest. Most notably, the crown area (6) has an outer diameter in continuity with that of the threaded body (5), the crown area (6) not being wider than the threaded body (5) (in other words, in qualitative terms, unlike conventional implants the implant-expander does not have a head, a head being understood as a crown-shaped ending that is wider than the threaded body). This enables the expansion to be controlled throughout the whole crest (which can be very narrow) in a uniform and very precise manner, without the risk of fracturing the bone crest completely. In addition, the crown area (6) comprises an anti-rotational blind hole (8) for the insertion of a torque-applying tool to enable the positioning and screwing of the implant-expander (3) in the bone crest.

Figures 4 and 5 show an embodiment of the implant-expander (3) according to the invention, which is narrower than the preceding one. A specific feature of which is the fact that the crown area (6) is not threaded in its entirety but has threaded outer walls with increasingly deep thread characteristics until they match those of the thread of the threaded body (5). This helps increase the thickness of the walls of the crown area (6) in the area of entrance to the blind hole (8), strengthening the implant-expander in this area and therefore reducing the risk of it breaking during its insertion in the bone crest.

Preferably, the threaded body (5) comprises a cylindrical threaded part (5a) closer to the crown area (6) and a threaded part with a decreasing diameter (5b) closer to the apex (7), in order to reduce the torque that must be applied on the implant in order to insert it in the bone crest, with the result that said insertion is performed gently and without effort. Said threaded part with a decreasing diameter (5b) may have a conical shape, such as the one shown in the embodiments of Figures 2 to 5.

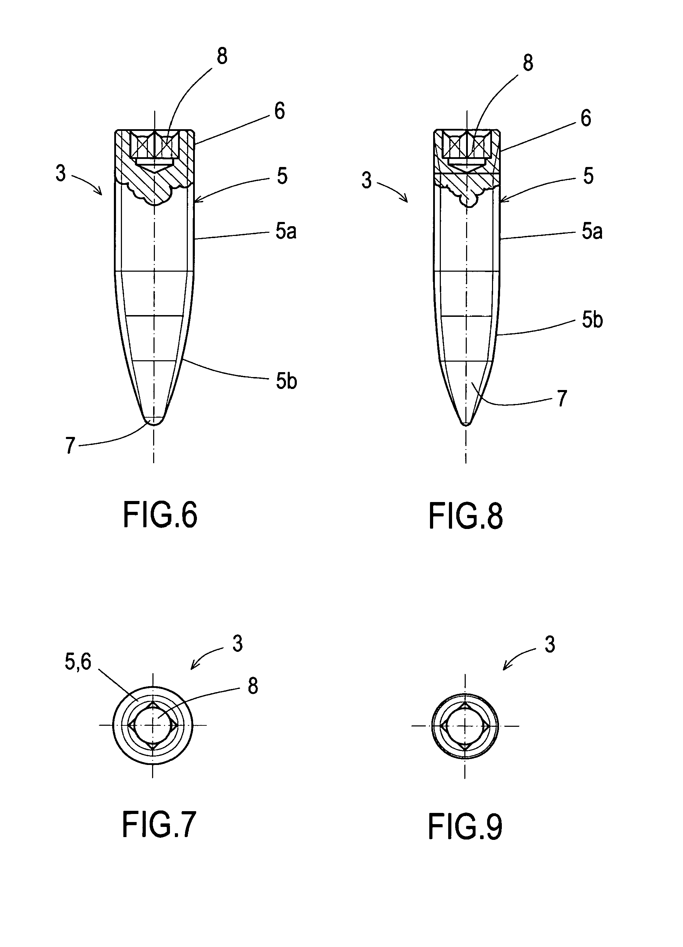

Figures 6 and 7 show a not claimed embodiment and figures 8 and 9 show an additional embodiment of the implant-expander (3) according to the invention, with the specific feature that in these embodiments the threaded part with a decreasing diameter (5b) has curved sides. In addition, in these embodiments the apex (7) is also threaded.

In terms of size, the cylindrical threaded part (5a) of the transitional dental implant (3) has a diameter ranging between 2 and 3.5 mm. This small diameter allows the implant-expander to carry out a widening process that is both gradual and free-of-risk to the bone crest, even in bone crests of a very small width in which the carrying out of a conventional process for expanding bone crest is unpredictable.

## Claims

1. Implant-expander (3), for expanding the bone crest (1) of a patient for the purpose of receiving the insertion of a final dental implant (4), said implant-expander (3) being manufactured from a biocompatible and osteoconductive material and comprises a threaded body (5), a crown area (6) and an apex (7), the crown area (6) having an outer diameter in continuity with that of the threaded body (5), the crown area (6) not being wider than the threaded body (5), the crown area (6) further comprising an anti-rotational blind hole (8) for the insertion of a torque applying tool, **characterised in that** the crown area (6) has outer walls that are partly threaded, and **in that** the outer walls of the crown area (6) have increasingly deep thread characteristics until they match those of the thread of the threaded body (5).

2. Implant-expander (3) according to claim 1, **characterised in that** the implant-expander (3) is manufactured from titanium.

3. Implant-expander (3) according to claim 2, **characterised in that** the implant-expander (3) is manufactured from grade 5 titanium (Ti₆Al₄V).

4. Implant-expander (3) according to claim 2, **characterised in that** the implant-expander (3) has a rough surface obtained by surface treatment with acids.

5. Implant-expander (3) according to claim 1, **characterised in that** the threaded body (5) comprises a cylindrical threaded part (5a) closer to the crown area (6) and a threaded part with a decreasing diameter (5b) closer to the apex (7).

6. Implant-expander (3) according to claim 5, **characterised in that** the threaded part with a decreasing diameter (5b) is conical.

7. Implant-expander (3) according to claim 5, **characterised in that** the threaded part with a decreasing diameter (5b) has curved sides.

8. Implant-expander (3) according to claim 1, **characterised in that** the apex (7) is threaded.

9. Implant-expander (3) according to claim 1, **characterised in that** the cylindrical threaded part (5a) has a diameter of between 2 and 3.5 mm.

## Patentansprüche

1. Implantat-Expander (3) für die Erweiterung des Knochenkamms eines Patienten zur Aufnahme eines endgültigen Zahnimplantats (4), wobei der besagte Implantat-Expander (3) aus einem biokompatiblem und osteokonduktivemMaterial gefertigtist und einen Gewindekörper (5), einen Kronenbereich (6)und einenApex (7) umfasst,wobei der Kronenbereich (6) einen Außendurchmesser in Fortführung mit dem des Gewindekörpers (5) hat, derKronenbereich (6) nicht breiter als der Gewindekörper(5) ist, der Kronenbereich (6)ferner einverdrehgesichertes Sackloch (8)zum Einfügeneines Verdrehwerkzeugs umfasst, **dadurch gekennzeichnet, dass** der Kronenbereich(6) teilweise gewindete Außenwände hat und dass die Außenwände des Kronenbereichs (6) bis zur Übereinstimmung mit denen des Gewinde des Gewindekörpers (5)zunehmendtiefe Gewindeeigenschaften haben.

2. Implantat-Expander (3) nach Anspruch1, **dadurch gekennzeichnet**, dassderimplantat-Expander (3) aus Titan gefertigt ist.

3. Implantat-Expander (3) nach Anspruch 2, **dadurch gekennzeichnet, dass** der Implantat-Expander (3) aus Titan Grad 5 (TisAUV) gefertigt ist.

4. Implantat-Expander (3) nach Anspruch 2, **dadurch gekennzeichnet, dass** der Implantat-Expander (3) eine durch Oberflächenbehandlung mit Säuren gebildete raue Oberfläche hat.

5. Implantat-Expander (3) nach Anspruch1, **dadurch gekennzeichnet**, dassderGewindekörper (5) ein zylindrisches Gewindeteil (5a) näher am Kronenbereich (6) und ein Gewindeteil mit einem abnehmenden Durchmesser (5b) näher am Apex (7) umfasst.

6. Implantat-Expander (3) nach Anspruch5, **dadurch gekennzeichnet**, dassdasGewindeteil mit einem abnehmenden Durchmesser (5b) konisch ist.

7. Implantat-Expander (3) nach Anspruch 5, **dadurch gekennzeichnet, dass** das Gewindeteil mit einem abnehmendem Durchmesser (5b) gekurvte Seiten hat.

8. Implantat-Expander (3) nach Anspruch 1, **dadurch gekennzeichnet, dass** der Apex (7) gewindet ist.

9. Implantat-Expander (3) nach Anspruch 1, **dadurch gekennzeichnet, dass** daszylindrische Gewindeteil (5a) einen Durchmesser von 2 bis 3,5 mm hat.

## Revendications

1. Implant-expanseur (3), visant à élargir la crête osseuse (1) d'un patient afin de pouvoir y insérer ensuite un implant dentaire (4), leditimplant-expanseur (3) étantfabriqué à partir d'un matériau biocompatible et ostéoconducteur, et comprenant un corps fileté (5), une zone coronaire (6) et un sommet (7),la zone coronaire (6) ayant un diamètre extérieur en continuité avec lediamètre du corps fileté (5),la zone coronaire (6) n'étant pasplus large que le corps fileté (5),la zone coronaire (6) comprenant en outre un trou borgne anti-rotation (8) pour l'insertion d'un outil applicateur de couple, **caractérisé par le fait que** la zone coronaire(6) est dotée de parois extérieures partiellement filetées et **par le fait que** les parois extérieures de la zone coronaire (6) ont de plus en plus profonde caractéristiques de filetage jusqu'à ce qu'ils égalent ceux du filetage du corps fileté (5).

2. Implant-expanseur (3),selon la revendication 1,**caractérisé par le fait que** l'implant-expanseur (3) est fabriqué en titane.

3. Implant-expanseur (3),selon la revendication 2, **caractérisé par le fait que** l'implant-expanseur (3) est fabriqué en titane degré 5 (Ti₆Al₄V).

4. Implant-expanseur (3),selon la revendication 2, **caractérisé par le fait que** l'implant-expanseur (3) possède une surface rugueuse obtenue au moyen d'un traitement de surface à base d'acides.

5. Implant-expanseur (3),selon la revendication 1, **caractérisé par** le fait quele corps fileté (5) comprend une partie filetée cylindrique (5a) plus proche de la zone coronaire (6)et une partie filetée dotée d'un diamètre décroissant (5b) plus proche du sommet (7).

6. Implant-expanseur (3),selon la revendication 5, **caractérisé par le fait que** la partie filetée au diamètre décroissant (5b)est conique.

7. Implant-expanseur (3),selon la revendication 5, **caractérisé par** le fait quela partie filetée au diamètre décroissant (5b)est dotée de parois courbées.

8. Implant-expanseur (3),selon la revendication 1, **caractérisé par le fait que** le sommet (7)est fileté.

9. Implant-expanseur (3),selon la revendication 1, **caractérisé par le fait que** la zone filetée cylindrique (5a) aun diamètre compris entre 2et 3,5 mm.
